# EUROPÄISCHE PATENTANMELDUNG

(11) **EP 4 303 208 A1**
(43) Veröffentlichungstag der Anmeldung: **10.01.2024**
(21) Anmeldenummer: 22183347.8
(22) Anmeldetag: 06.07.2022
(51) Int. Cl.: C07C 45/50, C07C 47/347, C07C 29/141, C07C 31/27

(54) **VERFAHREN ZUR HERSTELLUNG VON DICIDAL**

(71) Anmelder: Evonik Oxeno GmbH & Co. KG, 45772 Marl (DE)
(72) Erfinder: FRANKE, Robert, 45772 Marl (DE); SCHNEIDER, Carolin, 40789 Monheim am Rhein (DE); JACKSTELL, Ralf, 18106 Rostock (DE); BELLER, Matthias, 18211 Ostseebad Nienhagen (DE); REUSCH, Dieter, 45772 Marl (DE); HÄGER, Harald, 59348 Lüdinghausen (DE)
(74) Vertreter: Evonik Patent Association

(57) **Zusammenfassung**

Verfahren zur Herstellung von Dicidal.

## Beschreibung

Die vorliegende Erfindung betrifft ein Verfahren zur Herstellung von Dicidal.

In US 2009/0171125 A1 wird ein Verfahren zur Hydroformylierung von cyclischen Olefinen beschrieben. Hierbei kommt ein Rh-Katalysator zum Einsatz.

Der vorliegende Erfindung lag die Aufgabe zugrunde, ein neues Hydroformylierungsverfahren bereitzustellen. Das Verfahren soll hierbei eine gegenüber der aus dem Stand der Technik bekannten Methode gesteigerte Ausbeute liefern.

Diese Aufgabe wird gelöst durch ein Verfahren gemäß Anspruch 1.

Verfahren umfassend die Verfahrensschritte:
a) Vorlegen von Dicyclopentadien;
b) Zugabe einer Verbindung gemäß der Formel (**I**): 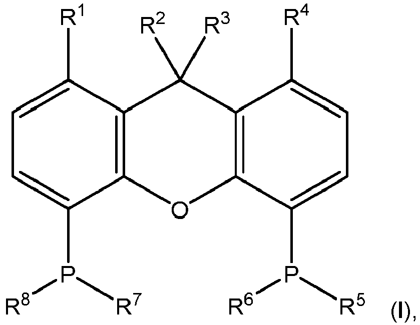 wobei R¹, R², R³, R⁴, R⁵, R⁶, R⁷, R⁸ ausgewählt sind aus: -H, -(C₁-C₁₂)-Alkyl, -Ph;
c) Zugabe einer Pt-Verbindung, welche zur Ausbildung eines Komplexes befähigt ist;
d) Zugabe einer Iod-Verbindung;
e) Zuführen von CO und H₂;
f) Erwärmen des Reaktionsgemisches aus a) bis e), wobei das Dicyclopentadien zu Dicidal umgesetzt wird.

Hierbei können die Verfahrensschritte a) bis e) in beliebiger Reihenfolge erfolgen. Üblicherweise erfolgt die Zugabe von CO und H₂ jedoch, nachdem die Reaktionspartner in den Schritten a) bis d) vorgelegt wurden.

Hierbei können die Verfahrensschritte c) und d) auch in einem Schritt, beispielsweise durch Zugabe von PtI₂, erfolgen.

In einer Variante des Verfahrens erfolgt die Zugabe der Pt-Verbindung und der Iod-Verbindung in einem Schritt, durch Zugabe von PtI₂.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, iso-Butyl, sec-Butyl, *tert*-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

In einer Variante des Verfahrens stehen R¹ und R⁴ für -H.

In einer Variante des Verfahrens stehen R⁵, R⁶, R⁷, R⁸ für -Ph.

In einer Variante des Verfahrens stehen R² und R³ für -(C₁-C₁₂)-Alkyl.

In einer Variante des Verfahrens stehen R² und R³ für -CH₃.

In einer Variante des Verfahrens weisen die Verbindung (**I**) die Struktur (1) auf:

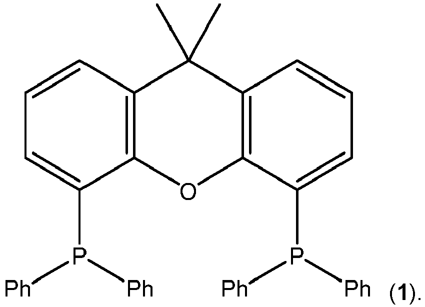

In einer Variante des Verfahrens ist die Pt-Verbindung ausgewählt aus: Pt(II)I₂, Pt(IV)I₄, Diphenyl(1,5-COD)Pt(II), Pt(II)(acac)₂, Pt(0)(PPh₃)₄, Pt(0)(DVTS)-Lösung (CAS: 68478-92-2), Pt(0)(Ethylen)(PPh₃)₂, Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂

In einer Variante des Verfahrens ist die Pt-Verbindung ausgewählt aus: Pt(II)I₂, Pt(II)(acac)₂ .

In einer Variante des Verfahrens ist die Pt-Verbindung Pt(II)I₂.

In einer Variante des Verfahrens ist die Iod-Verbindung ausgewählt aus: Alkalihalogenid, Erdalkalihalogenid, NH₄X, Alkylammoniumhalogenid, Dialkylhalogenid, Trialkylhalogenid, Tetraalkylhalogenid, Cycloalkylammoniumhalogenid.

In einer Variante des Verfahrens ist die Iod-Verbindung ausgewählt aus: Pt(II)I₂, Lil.

In einer Variante des Verfahrens wird PtI₂ in einer Menge zugegeben, gemessen in mol% bezogen auf Dicyclopentadien, so dass der Wert im Bereich von 0,1 mol% bis 5 mol% liegt.

In einer Variante des Verfahrens wird PtI₂ in einer Menge zugegeben, gemessen in mol% bezogen auf Dicyclopentadien, so dass der Wert im Bereich von 0,1 mol% bis 3 mol% liegt.

In einer Variante des Verfahrens wird PtI₂ in einer Menge zugegeben, gemessen in mol% bezogen auf Dicyclopentadien, so dass der Wert im Bereich von 0,1 mol% bis 1 mol% liegt.

In einer Variante des Verfahrens umfasst dieses den zusätzlichen Verfahrensschritt e'): e') Zugabe eines Lösungsmittels.

In einer Variante des Verfahrens ist das Lösungsmittel ausgewählt aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

In einer Variante des Verfahrens ist das Lösungsmittel ausgewählt aus: THF, DCM, ACN, Heptan, DMF, Toluol, Texanol.

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 1 MPa (20 bar) bis 6 MPa (50 bar).

In einer Variante des Verfahrens erfolgt das Erwärmen auf eine Temperatur im Bereich von 25 °C bis 150°C.

In einer Variante des Verfahrens erfolgt das Erwärmen auf eine Temperatur im Bereich von 30 °C bis 130°C.

In einer Variante des Verfahrens umfasst das Verfahren den zusätzlichen Verfahrensschritt g):
g) Umsetzen des Dicidals zu Dicidol.

In einer Variante des Verfahrens erfolgt die Umsetzung von Dicidal zu Dicidol unter Einsatz von "Shvos catalyst" (CAS 104439-77-2).

Im Folgenden soll die Erfindung anhand eines Ausführungsbeispiels näher erläutert werden.

### Versuchsbeschreibung

### Umsetzung von Dicyclopentadien zu Dicidal

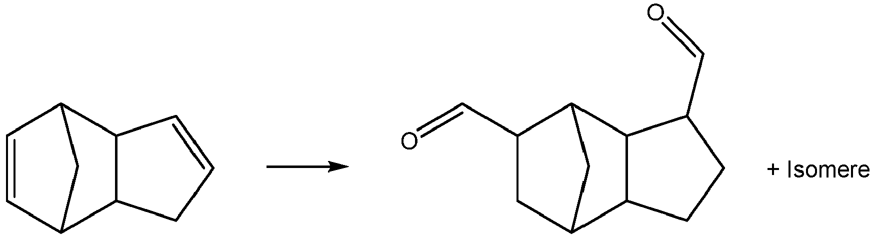

10 mmol Dicyclopentadien (DCPD), 10 ml absolutes Toluol, 0,5 mol% PtI₂, 2,2 Äquivalente Xantphos (1) (bezogen auf Pt) werden unter Argon in einen 25 ml Stahlautoklav der Firma Parr Instruments gegeben. Es werden 40 bar Synthesegas (CO/H₂ = 1:1) aufgepresst und die Reaktion durch Aufheizen auf 80 °C und Rührung gestartet. Diese Reaktion wird für 18,5 h bei 40 bar / 80 °C durchgeführt. Anschließend wird der Autoklav abgekühlt, der Druckabgelassen und eine GC Probe genommen.

In einem Vergleichsversuch wurden anstelle von PtI₂ Rh(acac)(CO)₂ hinzugegeben.

### Ausbeute Dicidal:

PtI₂: 89,5 %
Rh(acac)(CO)₂: < 5 %

### Umsetzung von Dicidal zu Dicidol

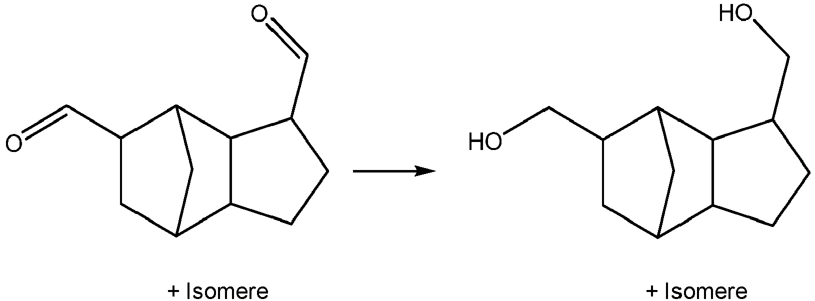

10 ml Toluol, 5 ml Ethanol, 5,5 g Dicidal (28,6 mmol) und 62,16 mg "Shvos catalyst" (CAS 104439-77-2) (0,2 mol% bezüglich Dicidal) werden in einem 100 ml Parr Druckautoklaven unter Argon mit 40 bar Wasserstoff unter Rührung auf 100 °C 20 Stunden erhitzt und zur Reaktion gebracht. Anschließend wird die Reaktion gestoppt, der Druck abgelassen und die Reaktionsmischung im Feinvakuum destilliert. Es wird ein farbloses Öl der Isomerenmischung von Dicidol als Fraktion bei 140 °C erhalten.

### Ausbeute Dicidol: 85 %

Wie die Versuchsergebnisse zeigen, wird die Aufgabe durch das erfindungsgemäße Verfahren gelöst.

## Patentansprüche

1. Verfahren umfassend die Verfahrensschritte:
a) Vorlegen von Dicyclopentadien;
b) Zugabe einer Verbindung gemäß der Formel (**I**): 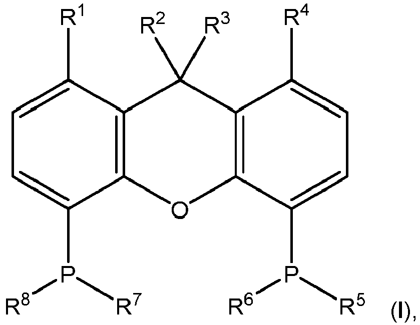 wobei R¹, R², R³, R⁴, R⁵, R⁶, R⁷, R⁸ ausgewählt sind aus: -H, -(C₁-C₁₂)-Alkyl, -Ph;
c) Zugabe einer Pt-Verbindung, welche zur Ausbildung eines Komplexes befähigt ist;
d) Zugabe einer Iod-Verbindung;
e) Zuführen von CO und H₂;
f) Erwärmen des Reaktionsgemisches aus a) bis e), wobei das Dicyclopentadien zu Dicidal umgesetzt wird.

2. Verfahren nach Anspruch 1,
wobei R¹ und R⁴ für -H stehen.

3. Verfahren nach einem der Ansprüche 1 oder 2,
wobei R⁵, R⁶, R⁷, R⁸ für -Ph stehen.

4. Verfahren nach einem der Ansprüche 1 bis 3,
wobei R² und R³ für -(C₁-C₁₂)-Alkyl stehen.

5. Verfahren nach einem der Ansprüche 1 bis 4,
wobei R² und R³ für -CH₃ stehen.

6. Verfahren nach einem der Ansprüche 1 bis 5,
wobei die Verbindung (**I**) die Struktur (1) aufweist:

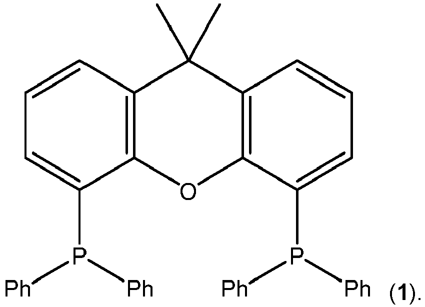

7. Verfahren nach einem der Ansprüche 1 bis 6,
wobei die Pt-Verbindung ausgewählt ist aus: Pt(II)I₂, Pt(IV)14, Diphenyl(1,5-COD)Pt(II), Pt(II)(acac)₂, Pt(O)(PPh₃)₄, Pt(0)(DVTS)-Lösung (CAS: 68478-92-2), Pt(O)(Ethylen)(PPh₃)₂, Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂

8. Verfahren nach einem der Ansprüche 1 bis 7,
wobei die Iod-Verbindung ausgewählt ist aus: Pt(II)I₂, Lil.

9. Verfahren nach einem der Ansprüche 1 bis 8,
wobei PtI₂ in einer Menge zugegeben wird, gemessen in mol% bezogen auf Dicyclopentadien, so dass der Wert im Bereich von 0,1 mol% bis 5 mol% liegt.

10. Verfahren nach einem der Ansprüche 1 bis 9,
umfassend den zusätzlichen Verfahrensschritt e'):
e') Zugabe eines Lösungsmittels.

11. Verfahren nach einem der Ansprüche 1 bis 10,
wobei das Zuführen von CO und H₂ bei einem Druck erfolgt in einem Bereich von 1 MPa (10 bar) bis 6 MPa (60 bar).

12. Verfahren nach einem der Ansprüche 1 bis 11,
wobei das Erwärmen auf eine Temperatur erfolgt im Bereich von 25 °C bis 150°C.

13. Verfahren nach einem der Ansprüche 1 bis 12,
wobei das Verfahren den zusätzlichen Verfahrensschritt g) umfasst:
g) Umsetzen des Dicidals zu Dicidol.

14. Verfahren nach Anspruch 13,
wobei die Umsetzung von Dicidal zu Dicidol unter Einsatz von "Shvos catalyst" (CAS 104439-77-2) erfolgt.
